# EUROPEAN PATENT SPECIFICATION

(11) **EP 2 182 054 B1**
(45) Date of publication and mention of the grant of the patent: **13.07.2016**
(21) Application number: 09013063.4
(22) Date of filing: 15.10.2009
(51) Int. Cl.: C12N 5/077

(54) **Process for identification and isolation of mesenchymal cells with marked bone regenerative ability**
Verfahren zur Aufreinigung und Isolierung mesenchymaler Zellen mit deutlicher Knochenregenerationsfähigkeit
Proces pour l'isolation et la purification de cellules mesenchymateuses avec une capacité élevé de régénération osseuse.

(30) Priority: 16.10.2008 IT MI20081831
(43) Date of publication of application: 05.05.2010
(73) Proprietor: Universita' Degli Studi di Bari, 70121 Bari (IT)
(72) Inventor: Crovace, Antonio, 70010 Valenzano (BA) (IT); Francioso, Edda Giuseppina, 70010 Valenzano (BA) (IT); Alessandri, Giulio, 20133 Milano (IT)
(74) Representative: Trupiano, Federica

(56) References cited:
- WO-A-97/40137
- XIA Z ET AL: "Efficient characterisation of human cell-bioceramic interactions in vitro and in vivo by using enhanced GFP-labelled mesenchymal stem cells" BIOMATERIALS, ELSEVIER SCIENCE PUBLISHERS BV., BARKING, GB, vol. 26, no. 29, 1 October 2005 (2005-10-01), pages 5790-5800, XP025280130 ISSN: 0142-9612 [retrieved on 2005-10-01]
- OLIVO CRISTINA ET AL: "In vivo bioluminescence imaging study to monitor ectopic bone formation by luciferase gene marked mesenchymal stem cells" JOURNAL OF ORTHOPAEDIC RESEARCH, vol. 26, no. 7, July 2008 (2008-07), pages 901-909, XP002535795 ISSN: 0736-0266
- SHIN M ET AL: "In vivo bone tissue engineering using mesenchymal stem cells on a novel electrospun nanofibrous scaffold" TISSUE ENGINEERING, LARCHMONT, NY, US, vol. 10, no. 1-2, 1 January 2004 (2004-01-01), pages 33-41, XP002389116 ISSN: 1076-3279
- BJERRE L ET AL: "Flow perfusion culture of human mesenchymal stem cells on silicate-substituted tricalcium phosphate scaffolds" BIOMATERIALS, ELSEVIER SCIENCE PUBLISHERS BV., BARKING, GB, vol. 29, no. 17, 1 June 2008 (2008-06-01), pages 2616-2627, XP022606545 ISSN: 0142-9612 [retrieved on 2008-03-28]
- PAPADIMITROPOULOS A ET AL: "Kinetics of in vivo bone deposition by bone marrow stromal cells within a resorbable porous calcium phosphate scaffold: An X-ray computed microtomography study" BIOTECHNOLOGY AND BIOENGINEERING, vol. 98, no. 1, September 2007 (2007-09), pages 271-281, XP002535794 ISSN: 0006-3592
- MASTROGIACOMO ET AL: "Engineering of bone using bone marrow stromal cells and a silicon-stabilized tricalcium phosphate bioceramic: Evidence for a coupling between bone formation and scaffold resorption" BIOMATERIALS, ELSEVIER SCIENCE PUBLISHERS BV., BARKING, GB, vol. 28, no. 7, 22 December 2006 (2006-12-22), pages 1376-1384, XP005812894 ISSN: 0142-9612
- GIANNONI P ET AL: "Regeneration of large bone defects in sheep using bone marrow stromal cells" JOURNAL OF TISSUE ENGINEERING AND REGENERATIVE MEDICINE, vol. 2, no. 5, July 2008 (2008-07), pages 253-262, XP002535796 ISSN: 1932-6254
- MURPHY J M ET AL: "Stem cell therapy in a caprine model of osteoarthritis" ARTHRITIS AND RHEUMATISM, LIPPINCOTT, PHILADELPHIA, US, vol. 48, no. 12, 1 December 2003 (2003-12-01), pages 3464-3474, XP002310438 ISSN: 0004-3591
- HOLT G E ET AL: "Evolution of an in vivo bioreactor" JOURNAL OF ORTHOPAEDIC RESEARCH, JOHN WILEY & SONS, INC, NEEDHAM, MA, vol. 23, no. 4, 1 July 2005 (2005-07-01), pages 916-923, XP004977057 ISSN: 0736-0266
- MASTROGIACOMO MADDALENA ET AL: "Reconstruction of extensive long bone defects in sheep using resorbable bioceramics based on silicon stabilized tricalcium phosphate" TISSUE ENGINEERING, LARCHMONT, NY, US, vol. 12, no. 5, 1 May 2006 (2006-05-01), pages 1261-1265, XP009091298 ISSN: 1076-3279
- VANESSA FRITZ ET AL: 'Antitumoral Activity and Osteogenic Potential of Mesenchymal Stem Cells Expressing the Urokinase-Type Plasminogen Antagonist Amino-Terminal Fragment in a Murine Model of Osteolytic Tumor' STEM CELLS vol. 26, no. 11, 28 August 2008, pages 2981 - 2990, XP055040567 DOI: 10.1634/stemcells.2008-0139 ISSN: 1066-5099
- XIAODONG GUO ET AL: "Bone regeneration with active angiogenesis by basic fibroblast growth factor gene transfected mesenchymal stem cells seeded on porous [beta]-TCP ceramic scaffolds; Bone regeneration with active angiogenesis by basic fibroblast growth factor gene transfected mesenchymal stem cells", BIOMEDICAL MATERIALS, INSTITUTE OF PHYSICS PUBLISHING, BRISTOL, GB, vol. 1, no. 3, 1 September 2006 (2006-09-01), pages 93-99, XP020111454, ISSN: 1748-605X, DOI: 10.1088/1748-6041/1/3/001

## Description

### STATE OF THE ART

The present invention concerns a process for obtaining mesenchymal cells with high bone tropism and marked bone regenerative ability.

In medical practice the problems and limits connected with the reconstruction and regeneration of bone tissue following traumas or pathologies of various types, in particular neoplasias, for example, are known.

Together with the cartilage, the bone tissue is part of the connective tissue specialised in the function of support. The bone tissue belongs to the connective tissue due both to its origin from the mesenchyma, the embryonal tissue acting as a matrix for all the connective tissues, and to its constitution, since it is formed of cells and intercellular substance composed of collagen fibres and fundamental anhistic substance. The peculiarity of bone tissue is that it is mineralised: in fact the intercellular substance is for the most part impregnated with mineral crystals, mainly calcium phosphate. The presence of minerals and the abundance and particular distribution as well of the organic components of the intercellular substance give this tissue marked mechanical properties of hardness and resistance to pressure, traction and torsion. Due to these properties, bone tissue constitutes an ideal material for formation of the bones of the skeleton, which constitute as a whole the supporting framework of the organism. Furthermore, given the considerable content of calcium salts, bone tissue represents the main deposit of calcium ion for metabolic needs of the whole organism. The deposition of calcium in the bone and its mobilisation, finely controlled by the endocrine mechanisms, contribute substantially to regulation of the plasmatic levels of this ion.

The cells of the bone tissue can be morphologically divided into 4 types: the *osteoprogenitor cells* (also called *preosteoblasts*), the *osteoblasts,* the *osteocytes* and the *osteoclasts.* Of these, osteoprogenitor cells, osteoblasts and osteocytes are in reality consecutive functional phases of the same cellular type, in its turn derived from the osteogenic differentiation of the pluripotent mesenchymal cell of the connective tissues, and can therefore be considered as autochthonous cells of the bone. The osteoclasts, on the other hand, derive from precursors that have migrated into the bone tissue from the blood, the so-called preosteoclasts, which in their turn differ from the stem cells of the haemopoietic bone marrow.

The osteoprogenitor cells have proliferative capacity which is manifested in particular during body growth but can also be active during adult life. When they begin to differentiate, the osteoprogenitor cells are transformed into osteoblasts. The osteoblasts are the cells primarily responsible for synthesis of the intercellular bone substance and its mineralisation.

The osteoblast is the site for synthesis of the organic molecules of the intercellular bone substance, which are subsequently secreted and assembled outside the cell. The osteocytes are the cells typical of mature bone, responsible for maintaining it and also able to initiate readaptation. They are terminal cells with a finite life, finely regulated by endocrine mechanisms.

The osteoclasts are the cells responsible for bone resorption. As already mentioned, they are not autochthonous cells of the bone tissue, since they do not belong to the line deriving from the osteoprogenitor cells. The precursors of the osteoclasts, called *preosteoclasts,* originate in the haemopoietic bone marrow and are related to the differentiative line of a category of white blood cells, the monocytes. The preosteoclasts are transported by the circulation to the sites where the bone resorption processes take place; from here, they migrate into the bone tissue and blend together giving rise to the active osteoclasts, syncytial elements able to dissolve the mineral component and enzymatically digest the organic components of the bone tissue.

Due to the characteristics described above, it is evident that the repair or restoration of the functions of said tissue is particularly difficult.

Bone healing can be schematised according to the following three mechanisms:
- *Osteogenesis:* this is the physiological process of production of new bone by the osteoblasts present on the margins of the interruption and therefore represents the mechanism via which normal fractures are healed without loss of substance.
- *Osteoconduction:* in this type of healing, the grafts of compact bone and some alloplastic implants function as scaffold, permitting the slow transfer of osteoblastic elements from the donor site together with the neoformation of vessels, with progressive colonisation of the bone gap and consequent healing.
- *Osteoinduction:* this is the physiological process of recruitment of immature cells and their differentiation into preosteoblasts, which takes place in all types of bone healing.

In relation to the type and entity of the damage, the use of biomaterials is known, i.e. materials interfacing with the biological systems in order to treat, increase or replace any tissue or organ of the organism.

A double interaction occurs between the biomaterial and the receiver organism: the biomaterial provokes a biological response of the organism, which in its turn causes a process of degradation in the biomaterial itself. The interactions take place at different levels: physical-chemical, molecular and cellular.

The operating environment of a biomaterial is the physiological environment, characterised by a considerable chemical activity and a high interval of mechanical stresses. The biomaterials are in direct contact with the biological fluids, i.e. water with ions in solution and co-presence of enzymes, proteins and cells. The physical-chemical conditions (pH, temperature) are practically constant over time, and this affects the design and "life" of a biomaterial.

A fundamental concept as regards biomaterials is biocompatibility, i.e. the ability of a material to act determining an appropriate response of the host in a given application.

All materials of various compositions that have acceptable biocompatibility characteristics are therefore defined as biomaterials. The concept of biocompatibility refers to the set of properties which a material must possess in order to be used safely in a biological organism. In fact the biological tissues react in various ways to introduction to or contact with a foreign body, depending on the physical-chemical and mechanical characteristics of the material.
The ideal material is not yet "available" and consequently the materials used so far are those with the best biocompatibility characteristics. Some of the characteristics which a biocompatible material must possess are, for example, the following:
- absence of carcinogenicity
- absence of immunogenicity
- absence of teratogenicity
- absence of toxicity

From a clinical point of view, bone grafts still represent the best model, since they provide the three fundamental components necessary for correct bone healing: osteoblasts, osteoconduction and osteoinduction. However the availability of autologous bone grafts is limited. In many clinical situations, therefore, alloplastic materials are used; these alone, however, are not able to provide all the necessary components for correct healing.

The known art therefore has various complications and limits which are overcome by the present invention.

### DESCRIPTION OF THE INVENTION

The present invention therefore concerns a process for obtaining mesenchymal cells. In particular the process according to the invention allows the development of an organ-specific cell therapy.

In general terms, as will be described in further detail below, the process subject of the invention provides for in vitro expansion, labelling and preparation in an appropriate manner of a pool of mesenchymal cells. In the vicinity of a bone lesion, at least one scaffold will be inserted and appropriately positioned, made of biocompatible material and particularly suited to facilitating bone reconstruction. The mesenchymal cells, as obtained above, are inoculated, and after a certain time has elapsed, the scaffold(s) is (are) removed. It has surprisingly been noted that the mesenchymal cells, as prepared according to the invention, are attracted by the scaffold with an automatic selection of those provided with marked bone regenerative ability. By removal of the scaffold it is possible to obtain a pool of highly selected mesenchymal cells provided with high bone regenerative ability, which could therefore be used in the processes of bone regeneration and reconstruction necessary as a result of various types of damage.

The process according to the invention therefore allows recruitment of the labelled adult mesenchymal cells at the site of the bone lesion where the scaffold has been grafted, due also to the attraction ability of the biomaterial which constitutes the scaffold. The pool of adult mesenchymal cells which reaches the graft site has particular bone regenerative properties.

More specifically, the process according to the invention comprises the following phases:
a) in vitro expanding mesenchymal cells;
b) labelling said mesenchymal cells with suitable expression vectors, obtaining labelled cells;
c) selecting said labelled cells that, after inoculation into the circulation system of an experimental model have been attracted by a scaffold positioned at the site of a bone lesion and subsequently removed
d) expanding said selected cells in culture to obtain a pool with high bone tropism.

The process according to the invention therefore advantageously allows obtainment of mesenchymal cells with marked bone regenerative ability from a heterogeneous pool of mesenchymal cells.

The cell population obtained via the process according to the invention has a marked bone tropism, and therefore offers the advantage of locating in a preferential and specific manner in said bone tissue.

In particular the process provides for:
Labelling adult, human or animal mesenchymal cells, derived from different tissues such as, for example, bone marrow, tendons, muscle or adipose tissue for example with a lentiviral vector engineered with a protein that makes them fluorescent and selectable through antibiotic treatment. Advantageously the lentiviral vector used and likewise the protein can be those known in the state of the art and even more advantageously, for example, the lentiviral vector is the one currently sold under the name Lentimax engineered with the Green Fluorescent Protein (GFP) and gene for antibiotic resistance to puromycin. Said Lentimax vector is a highly optimised lentiviral vector of the HIV virus, adapted to be engineered to express any gene of interest.

Creating the bone damage in an experimental model. A scaffold (or more than one according to the type of damage) or a biocompatible supporting material is inserted at the site of the lesion. Advantageously the material used at the site of the lesion is, for example, silicone tricalcium phosphate. This material, obtained from the combination of different materials like tricalcium phosphate and silicone, generates a new material with particularly advantageous technical characteristics. Said material is used in the treatment of pathologies and damage connected with bone loss. The use of ceramics, such as tricalcium phosphate, is known due to their osteointegration and osteoconduction properties, but this is not so for silicone. The latter is usually used in replacement for repairing damage to the soft tissues but not for treating pathologies or damages connected with bone loss. According to the present invention, however, silicone is used as a material for treating damage connected with bone loss. In the case of the present invention, therefore, said silicone in combination with the tricalcium phosphate is used in the case of bone lesion, where the bone has physiological characteristics such as hardness, compactness and resistance. It is therefore evident that the biocompatible material used has particular properties which distinguish it from those currently used in medical practice for the treatment of pathologies or damage similar or identical to those of the present invention. The ceramic component, or the tricalcium phosphate, due to its mineralisation properties and porosity, allows migration of osteogenic cells and allows vascular development. The non-carbonic polymeric component, i.e. the silicone, on the other hand could provide characteristics of osteoconduction. The intrinsic properties of the new material, such as size, morphology, crystallinity of the granules, porosity and composition, are also particularly advantageous. Said characteristics are particularly suited to the objects of the present invention.
Inoculating the cells thus obtained into the circulation system of the experimental models.
Removing the scaffolds used as a support at the site of the damage, and isolating cells of mesenchymal origin that have colonised the scaffolds and the bone lesion.
Selecting isolated cells, for example, for fluorescence under a microscope and placed in a culture in antibiotic selection to obtain a cell pool with high bone tropism.

The scaffolds are usually inserted in large experimental animal models of bone lesion, preferably sheep. Said scaffolds consist of biomaterial, in particular those made of silicone tricalcium phosphate are advantageously used. According to one of the experimental models evaluated, the osteoinductive scaffold is positioned inside a bone lesion made in the spongy tissue of a metacarpus in an experimental model. The biomaterial implanted has the function of attracting and recruiting the mesenchymal cells obtained according to the above.

The adult mesenchymal cells thus obtained unlike those obtained according to the protocols and the treatments known in the art, have a high bone regenerative ability even if isolated from a heterogeneous pool.

Therefore the engineered adult mesenchymal cells locate, with high tropism, in the damaged bone tissue, increasing the bone regenerative and reconstructive ability. Adult mesenchymal cells according to the above, are useful as targeted bone tissue cell therapy.

The adult mesenchymal cells obtained according to the above process can be advantageously used as cell therapy suitable for restoring the damaged bone tissue of a lesion or for use as a carrier of drugs to said bone tissue.

Preferably said adult mesenchymal cells can be used as a carrier for chemotherapy drugs in the treatment of neoplastic or metastatic bone lesions.

Said adult mesenchymal cells thus obtained can be administered by intramuscular, intravenous and all other known administration routes compatible with the product. Pharmaceutical compositions contain said adult mesenchymal cells provided with high tropism and obtained according to the process subject of the present invention, together with any excipients known in pharmaceutical practice.

The present invention is better illustrated via the non-limiting examples given below.

### EXAMPLE 1 - Experimental model of bone damage.

Scaffolds of silicone tricalcium phosphate were used for the study, with a porous structure similar to that of the spongy bone, in the form of small pads, e.g. with dimensions of (4 mm x 3 mm x 2 mm) with pore size varying between 200 and 500 µm. Said scaffolds were implanted in a hole made with a 6 mm drill in the spongy tissue of the medial distal portion of the metacarpus of the right-hand rear limb of sheep weighing 70 Kg live weight, previously sedated and anaesthetised. After incising the cutaneous and subcutaneous tissue, the bone surface was reached. After incising and detaching the periosteum, it was retracted to permit positioning of the guide and the drill for subsequent drilling of the spongy tissue. Once the tunnel had been obtained in the spongy tissue, it was washed with physiological solution and, using a spatula, 6 pads of biomaterial were inserted in the tunnel until it was totally filled. The periosteum was repositioned, and the subcutaneous tissue and the skin were sutured according to medical practice. The distal portion of the limb operated was medicated and bandaged and in the post-operative phase the animals were given antibiotic and analgesic treatment.

Three days after the implant, after application of Esmarch's bandage on the distal portion of the non-operated rear limb, the cells transfected and diluted in 35 ml of sterile saline solution were inoculated slowly, after intra-arterial cannulation of the metatarsal artery. Six days after the implant the animals were re-anaesthetised and operated on to remove the scaffolds previously positioned inside the tunnel in the metacarpus. The scaffolds then underwent digestion for recovery of the cells.

### EXAMPLE 2 - Protocol for treatment of engineered mesenchymal cells.

The adult mesenchymal cells deriving from the bone marrow taken by means of Jamshidi needles from the ala of the ilium of the sheep used for the experiment were transfected with the GFP lentiviral vector and kept in a culture in complete Coon's medium at 37°C in an atmosphere enriched with 5% of CO2.

### EXAMPLE 3 - Inoculation of engineered cells.

The engineered cells were inoculated arterially via the metacarpal artery of the limb not affected by the lesion. Said cells were inoculated in a quantity of approximately 500,000 cells labelled with GFP/Kg of body weight. The cells were diluted in 35 ml of physiological solution and before inoculation the animals were previously treated with sodium heparin (1 ml of a solution containing 5000 µi/ml).

### EXAMPLE 4 - Isolation and removal of the cell population in the animals surgically treated.

The implanted scaffolds were surgically removed and the cells that had colonised both the implants and the bone lesion were extracted therefrom. The biomaterial was recovered in sterile conditions, washed in phosphate buffer (PBS) and underwent digestion with type D collagenase and bovine albumin at 37°C under agitation. The digestion times with the enzyme were between 2h and 2h 30 minutes. The supernatant was centrifuged at 1800 rpm for 10 minutes. The cells recovered in this phase were then placed in culture in complete Coon's medium (foetal serum 10%, glutamine, penicillin/streptomycin). The cultures obtained were evaluated under the microscope in order to identify by fluorescence the number of labelled cells with high tropism. An antibiotic selection of the culture was performed with puromycin. The cells obtained were inoculated in animals previously treated as described in example 1 in order to evaluate the tropism.

## Claims

1. Process to obtain mesenchymal cells with marked bone regenerative ability, which includes the steps of: a) in vitro expanding isolated mesenchymal cells, b) labeling said mesenchymal cells with suitable expression vectors, obtaining labelled cells c) selecting said labelled cells that, after inoculation into the circulation system of an experimental model have been attracted by a scaffold positioned at the site of a bone lesion and subsequently removed d) expanding said selected cells in culture to obtain a cell pool with high bone tropism.

2. Process according to claim 1 wherein said isolated mesenchymal cells are adult human or animal mesenchymal cells, derived from tissues selected from bone marrow, tendons, muscle, adipose tissue and said labeling step b) of said adult mesenchymal cells is achieved with lentiviral vector engineered with a protein that makes them fluorescent and selectable through antibiotic treatment.

3. Process according to claim 1 and claim 2 **characterized in that** said labeling b) of said adult mesenchymal cells is achieved with the Lentimax lentiviral vector engineered with the protein Green Fluorescent Protein (GFP) and gene for antibiotic resistance to puromycin and said scaffold is a scaffold made of silicone tricalcium phosphate.

## Patentansprüche

1. Verfahren zur Herstellung von mesenchymalen Zellen mit markierter Knochenregenerationsfähigkeit, welches die folgenden Schritte umfasst:
a) In-vitro-Expandieren von isolierten mesenchymalen Zellen, b) Markieren der mesenchymalen Zellen mit geeigneten Expressionsvektoren, wobei markierte Zellen erhalten werden, c) Auswählen der markierten Zellen, die nach Inokkulieren in dem Zirkulationssystem eines experimentellen Modells durch ein Gerüst angezogen wurden, welches an der Stelle einer Knochenläsion positioniert ist, und danach entfernt wurden, d) Expandieren der ausgewählten Zellen in einer Kultur, um einen Zellenpool mit hohem Knochentropismus zu erhalten.

2. Verfahren gemäß Anspruch 1, wobei die isolierten mesenchymalen Zellen adulte humane oder tierische mesenchymale Zellen sind, welche von Geweben, welche aus Knochenmark-, Sehnen-, Muskel- und Fettgeweben ausgewählt sind, abgeleitet sind und der Markierungsschritt b) der adulten mesenchymalen Zellen mit einem lentiviralen Vektor erreicht wird, welcher mit einem Protein ausgestattet ist, welches diese fluoreszierend und durch Antibiotikabehandlung auswählbar macht.

3. Verfahren gemäß Anspruch 1 oder Anspruch 2, **dadurch gekennzeichnet, dass** das Markieren b) der adulten mesenchymalen Zellen mit dem Lentimax lentiviralen Vektor erreicht wird, welches mit dem Protein Grün fluoreszierendes Protein (GFP) und Gen für Antibiotikaresistenz auf Puromycin ausgestattet ist, und das Gerüst ein aus Silicium-Tricalciumphosphat hergestelltes Gerüst ist.

## Revendications

1. Procédé d'obtention de cellules mésenchymateuses dotées d'une capacité de régénération osseuse marquée, qui comprend les étapes suivantes :
a) l'expansion *in vitro* de cellules mésenchymateuses isolées, b) le marquage desdites cellules mésenchymateuses avec des vecteurs d'expression appropriés, l'obtention de cellules marquées, c) la sélection desdites cellules marquées qui, après inoculation dans le système circulatoire d'un modèle expérimental, ont été attirées par un échafaudage positionné au niveau du site d'une lésion osseuse et ensuite éliminé, d) l'expansion desdites cellules sélectionnées en culture pour obtenir un groupe de cellules présentant un tropisme osseux élevé.

2. Procédé selon la revendication 1, dans lequel lesdites cellules mésenchymateuses isolées sont des cellules mésenchymateuses humaines ou animales adultes, dérivées de tissus choisis parmi la moelle osseuse, les tendons, les muscles, le tissu adipeux et ladite étape de marquage b) desdites cellules mésenchymateuses adultes est obtenue avec un vecteur lentiviral modifié avec une protéine qui les rend fluorescentes et sélectionnables par l'intermédiaire d'un traitement antibiotique.

3. Procécé selon la revendication 1 et la revendication 2, **caractérisé en ce que** ledit marquage b) desdites cellules mésenchymateuses adultes est obtenu avec le vecteur lentiviral Lentimax modifié avec la protéine fluorescente verte (GFP) et le gène pour la résistance à un antibiotique pour la puromycine et ledit échafaudage est un échafaudage fait de phosphate tricalcique de silicone.
